## Europäisches Patentamt

(19) **European Patent Office**

**Office européen des brevets**

(11) Publication number : **0 341 291 B1**

(12) # EUROPEAN PATENT SPECIFICATION

(45) Date of publication of patent specification : **02.12.92 Bulletin 92/49**

(51) Int. Cl.[5] : **A61K 9/22**

(21) Application number : **89900112.7**

(22) Date of filing : **08.11.88**

(86) International application number : **PCT/US88/03988**

(87) International publication number : **WO 89/04654 01.06.89 Gazette 89/12**

(54) CONTROLLED ADMINISTRATION OF BENEFICIAL AGENT TO BLOOD.

(30) Priority : **16.11.87 US 120896**

(43) Date of publication of application : **15.11.89 Bulletin 89/46**

(45) Publication of the grant of the patent : **02.12.92 Bulletin 92/49**

(84) Designated Contracting States : **BE DE FR GB IT NL SE**

(56) References cited :
EP-A- 0 059 694
EP-A- 0 168 522
CH-A- 497 181
GB-A- 1 595 388
US-A- 25 129
US-A- 3 965 896
US-A- 4 086 924
US-A- 4 235 236
US-A- 4 257 426
US-A- 4 552 555
US-A- 4 758 430
Transfusion, vol. 24, no. 6, issued 1984
(Philadelphia), B.A. Myher et al.:
"Preservation of red cell antigens during
storageof blood with different anticoagulants,
see pages 499-501

(73) Proprietor : **BAXTER INTERNATIONAL INC. (a Delaware corporation)**
**One Baxter Parkway**
**Deerfield Illinois 60015 (US)**

(72) Inventor : **BACEHOWSKI, David, V.**
**33136 Lakeshore Drive**
**Wildwood, IL 60030 (US)**
Inventor : **MIRIPOL, Jeffrey, E.**
**1323 Greenleaf Street**
**Evanston, IL 60202 (US)**
Inventor : **NEEDHAM, Thomas, E.**
**1105 Heather Road**
**Deerfield, IL 60015 (US)**
Inventor : **KARTINOS, Nicholas, J.**
**1810 Woodland Avenue**
**Park Ridge, IL 60068 (US)**

(74) Representative : **MacGregor, Gordon et al ERIC POTTER & CLARKSON St. Mary's Court St. Mary's Gate Nottingham, NG1 1LE (GB)**

EP 0 341 291 B1

## Description

TECHNICAL FIELD

As blood is collected from the donor, it passes into a container such as a blood bag which contains an anticoagulant system. Such anticoagulant systems are typically a small amount of liquid solution which is stored in the bag and collection tubing, being typically ACD, CPD, CPD-adenine, or the like. Additionally, it has been suggested that the anticoagulant system may be placed as a dried coating or powder in the interior of the blood collection container.

With these systems, as the blood is introduced into the container, the ratio of the whole blood present to the anticoagulant present is undesirably low, consequently producing a significant and detrimental imbalance. While this problem is quickly corrected with the addition of subsequent amounts of blood, it can be damaging to the first aliquot of collected blood components.

Accordingly, there is a need for a blood handling system where beneficial agents such as anticoagulant/storage agents may be administered to the blood without the initial, undesirable osmotic shock effect that currently takes place at the beginning of the blood collection.

Additionally, in the manufacture of conventional blood storage bags which contain a liquid anticoagulant, a capital intensive and closely controlled manufacturing process is required, including a liquid filling operation (which requires a clean room), batch steam sterilization procedures, and packaging of the blood bag in an additional moisture barrier container for storage. If it were possible to make use of blood bags which did not contain a liquid anticoagulant system, it would be possible to greatly simplify the manufacturing process, making use of radiation sterilization, and eliminating the additional moisture barrier packing which is currently necessary.

Additionally, blood storage bags which contain either a liquid anticoagulant or a dried coating thereof on the interior walls result in a container that must be completely filled with blood in order to be usable. If, for any reason, the donor is unable to donate one complete unit of blood, the entire, underfilled unit typically is not used, due to an excess concentration of anticoagulation agent present in the reduced volume of blood, since the anticoagulant present in the bag is intended for one complete unit of blood. If empty, anticoagulant-free blood storage bags were used in which the blood was still properly provided with anticoagulant/storage agents, these underfilled units could still be used for pediatric or partial transfusion purposes, and would not have to be discarded.

In accordance with this invention, the above problems, and other problems as well, may be solved, to permit the application of a beneficial agent such as anticoagulant/storage agents to blood while the blood is passing through a conduit into a container. Thus, blood storage containers without any beneficial agent therein may be manufactured and used, for the significant manufacturing advantages described above. Also, because of this invention, partial units of blood may be collected and used.

Reference is made to GB-A-1595388. This document discloses a method of adding a coagulant agent to blood during collection from a donor comprising passing the blood through a chamber of a conduit across a supply of blood-soluble anticoagulant agent in said chamber. The agent is provided as a heparin coating on filaments of a filter, through which the blood is passed prior to collection.

The present invention is characterised in that said anticoagulant agent is provided in tablet form, the tablet having opposed flat major surfaces of greater dimension than the thickness of the tablet, in that the tablet is arranged in said chamber so as to provide flow passages between said major surfaces and the chamber wall, and in that the blood is passed along said flow passages across said major surfaces, whereby a controlled amount of agent is caused to enter said passing blood, whereby no substantial portion of said blood is exposed to a significantly higher concentration of said agent than other portions of said blood.

In the event that less than a unit of blood passes across the beneficial agent, less than the nornal amount of the beneficial agent will enter the blood, so that the partial unit of blood contains substantially the same concentration of beneficial agent that a whole unit of blood would contain, permitting its use. Additionally, in those preferred circumstances where the container is substantially free of the beneficial agent, but for the beneficial agent passing into the container with the blood, simplified manufacturing methods may be used for manufacturing the container.

The tablet may comprise a solid mass of dextrose molded or pressed into tablet form, and which contains an effective anticoagulating amount of a soluble, nontoxic citrate, so that the dextrose and the citrate dissolve together in a controlled release manner in the passing blood. Additionally, the anticoagulant agent formulation may include an amount of soluble, nontoxic phosphate and/or adenine, which is effective to promote the blood storage of the anticoagulant preparation. Examples of usable citrates and phosphates include sodium citrate, citric acid, and monosodium or disodium phosphate. Another usable anticoagulant may be heparin.

Additionally, other dry ingredients may be incorporated in the glucose mass, such as mannitol, adenine, or any other desired beneficial agent. Additionally, other media for providing the solid, blood-soluble mass may be used such as sodium citrate per se, which serves as an anticoagulant in its own right.

By the term "blood", it is intended to include not only whole blood, but components of blood as may be desired, for example blood plasma (either platelet rich or platelet poor) or suspensions of packed cells, white cells, or platelets in saline, any other appropriate suspension of blood cells, or the like.

## DESCRIPTION OF DRAWINGS

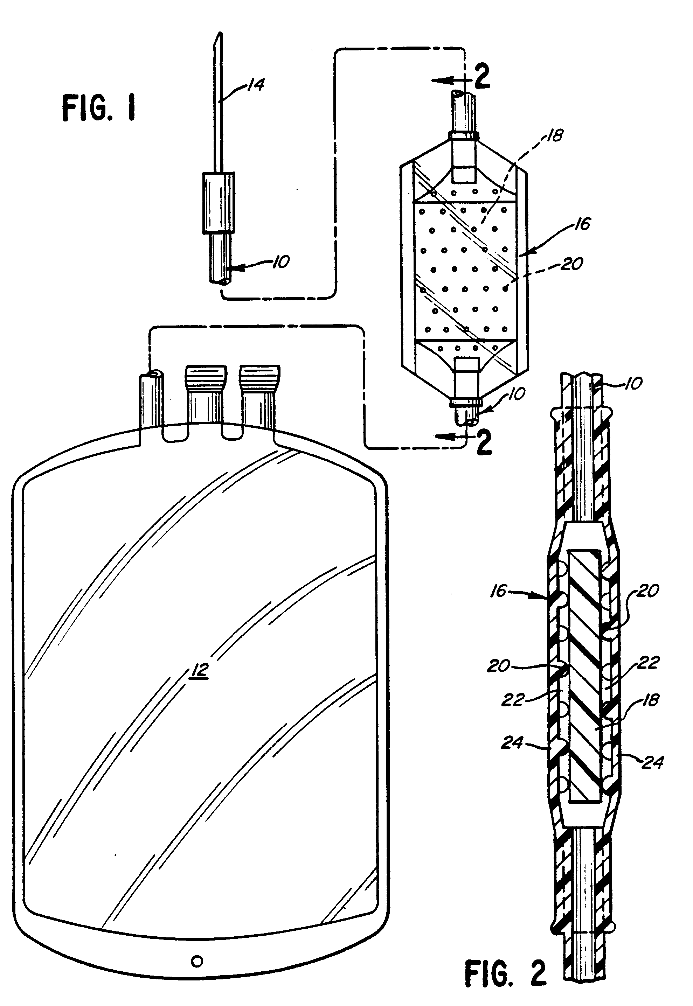

Fig. 1 is a plan view of a blood collecting set made in accordance with this invention, shown to be integrally connected to a blood storage bag.

Fig. 2 is a sectional view taken along line 2-2 of Fig. 1.

## DESCRIPTION OF SPECIFIC EMBODIMENT

Referring to the drawings, there is shown a set 10 for collecting blood from a patient or other donor and conveying it to blood storage container 12, which may be made in substantially conventional manner. Set 10 carries at one end a conventional blood connection needle 14 for collecting blood from the venous system of the donor, which blood then passes through set 10 into blood bag 12.

The conduit of set 10 defines an enlarged portion or chamber 16 which includes a solid mass 18 of dry blood storage anticoagulant preparation, typically having a water content on the order of no more than about 2% by weight. Anticoagulant tablet 18 is shown to be made in the form of a thin, rectangular member positioned within enlarged portion 16. The tablet 18 has opposed, flat major surfaces of greater dimension than the thickness of the tablet The inner walls of both sides of the chamber 16 define an array of small projections 20 which project inwardly to define flow passages between the inner walls of the chamber 16 and the major surfaces of the anticoagulant preparation tablet 18.

Specifically, anticoagulant preparation tablet 18 may comprise, for example, a dried mixture of:

|  | Weight % |
|---|---|
| Dextrose | 44 |
| Sodium Citrate | 46 |
| Citric Acid | 6 |
| Sodium Biphosphate | 4 |

Alternatively, another example of tablet 18 of this invention may be pressed together out of the following ingredients:

|  | Weight present | |
|---|---|---|
| Dextrose | 1.62 | gm. |
| Sodium citrate | 0.83 | gm. |
| Citric acid | 0.206 | gm. |
| Sodium Biphosphate | 0.140 | gm. |
| Mannitol – as a binding agent | 0.24 | gm. |
| Poly(ethylene glycol) – as a tablet lubricant | 2% of total wt. | |

Tablet 18 of either of the above mixtures of materials may be formed into a solid, pressed together matrix, or it may be mixed together with an aqueous vehicle, with the water being later evaporated away to form a glassy or microcrystalline composition.

The proportions of citric acid and sodium citrate, and their concentrations, are selected to achieve the desired concentration and pH for the passing blood.

Instead of citrate anticoagulant, the tablet 18 may alternatively include heparin anticoagulant, such as about 1800 to 2500 units of heparin for use with a single unit of whole blood. A single unit of whole blood is between about 400 to 500 ml. of liquid.

Blood bag 12 may be initially free of any anticoagulant. The entire system, including tablet 18, is typically radiation sterilizable, since it is substantially free of moisture. Additionally, no overpouch or other outer package is required to prevent the loss of water from a liquid anticoagulant system, which constitutes a significant advantage over the conventional present blood collection equipment.

For use, a conventional phlebotomy is made into a vein of a blood donor with needle 14, and the blood flows through conduit 10. Is it flows, it enters enlarged portion 16, where it flows in a pair of opposed flow halves 22 between the walls 24 of enlarged portion 16 and the flat surfaces of tablet 18. As the blood so flows, the substance of tablet 18 is dissolved away into the flowing blood at a relatively uniform rate of dissolution, so that each individual portion of the flowing blood passing through conduit 10 picks up a substantially similar amount of the dissolved substance of tablet 18 as it flows across it. From there, the flowing blood, carrying the dissolved anticoagulant, glucose, etc. passes into bag 12 for storage, without encountering an excessive concentration of anticoagulant or other agent.

It may be generally desirable for the tablet 18 to be of such a thickness that it is not completely eroded away by the time that the last of the blood has passed across it to fill bag 12 to its desired amount. When tablet 18 retains its basic rectangular shape to the end of the flow process, even though it becomes thinner, a relatively constant transfer of anticoagulant to all portions of the blood is achieved.

Any design of blood bag or other container may be utilized. If desired, the tablet 18 may be encapsulated and released via diffusion through a semipermeable membrane.

Additionally, the invention of this application may be used in conjunction with various other blood collection, separation, or handling procedures, for example plasma separation. Medications may be administered to blood by an apheresis procedure or the like.

## Claims

1. A method of adding an anticoagulant agent to blood during collection from a donor comprising passing the blood through a chamber (16) of a conduit across a supply of blood-soluble anticoagulant agent (18) in said chamber, characterised in that said anticoagulant agent is provided in tablet (18) form, the tablet having opposed flat major surfaces of greater dimension than the thickness of the tablet, in that the tablet is arranged in said chamber (16) so as to provide flow passages between said major surfaces and the chamber wall, and in that the blood is passed along said flow passages across said major surfaces, whereby a controlled amount of agent is caused to enter said passing blood, whereby no substantial portion of said blood is exposed to a significantly higher concentration of said agent than other portions of said blood.

2. The method of Claim 1, wherein the depth of each flow passage is less than the thickness of the tablet.

3. The method of Claim 1 or 2, in which the thickness of the tablet (18) is such that it is not completely eroded away by the time that the last of the blood being passed along said flow passages has passed across the tablet.

4. The method of Claim 1, 2 or 3, wherein said chamber wall defines projections (20) which project towards the tablet (18).

5. The method of any preceding Claim in which said blood is collected in a container (12) which is substantially free of said agent, but for agent passing into said container with said blood.

6. The method of any preceding Claim in which said anticoagulant agent is a suitable blood storage preparation.

7. The method of Claim 6 in which said tablet (18) is a solid mass of dextrose which contains an effective anticoagulating amount of soluble, nontoxic citrate.

8. The method of Claim 6 or 7 in which said tablet (18) includes an amount of soluble, nontoxic phosphate which is effective to promote the action of said blood storage anticoagulant agent.

9. The method of any preceding Claim in which said tablet (18) is in the form of adhering particles, and includes mannitol in sufficient amount to promote the adhesion of said particles to each other.

10. The method of any preceding claim in which said blood is whole blood.

11. A set for use in carrying out the method of Claim 1 and including a conduit having a chamber (16) containing a blood anticoagulant agent (18), characterised in that the anticoagulant agent is provided in tablet (18) form, the tablet having opposed flat major surfaces of greater dimension than the thickness of the tablet, in that the tablet is arranged in said chamber (16) so as to provide flow passages for blood between said major surfaces and the chamber wall.

12. The set of Claim 11, wherein the depth of each flow passage is less than the thickness of the tablet.

13. The set of Claim 11 or 12 in which said chamber wall defines projections (20) which project towards the tablet (18).

14. The set of claims 11, 12 or 13, in which said solid mass comprises dextrose which contains an effective anticoagulating amount of anticoagulant.

15. The set of any one of Claims 11 to 14, in which said solid mass is in the form of adhering particles, and includes mannitol in sufficient amount to promote the adhesion of said particles to each other.

16. The set of any one of Claims 11 to 15 having means (14) for communication with the venous system of a blood donor at one conduit end.

17. The set of any one of Claims 11 to 16 which is connected to a blood storage bag (12) at the other conduit end.

18. The set of any one of Claims 11 to 17 in which said anticoagulant agent comprises a soluble, nontoxic citrate.

19. The set of any one of Claims 11 to 17 in which said anticoagulant agent comprises heparin.

20. The set of Claim 18 in which said tablet (18) includes an amount of soluble, nontoxic phosphate which is effective to promote the action of said blood storage anticoagulant agent.

21. The set of any one of Claims 11 to 20 which has a conduit end connected to a blood storage bag (12), wherein the bag is essentially free of an anticoagulant preparation.

**Patentansprüche**

1. Verfahren zum Beimischen eines Antikoagulans zu Blut während der Entnahme von einem Spender, wobei das Verfahren folgendes aufweist: Leiten des Bluts durch eine Kammer (16) einer Leitung über einen Vorrat von blutlöslichem Antikoagulans (18) in der Kammer,
   dadurch gekennzeichnet,
   daß das Antikoagulans in Tablettenform (18) vorgesehen ist, wobei die Tablette gegenüberliegende ebene Hauptflächen mit größeren Dimensionen als die Dicke der Tablette hat, daß die Tablette in der Kammer (16) angeordnet ist, um Durchflußkanäle zwischen den Hauptflächen und der Kammerwand zu bilden, und daß das Blut entlang den Durchflußkanälen über die Hauptflächen geleitet wird, wodurch bewirkt wird, daß eine kontrollierte Menge von Antikoagulans in das vorbeiströmende Blut eintritt, so daß kein wesentlicher Teil des Bluts einer bedeutend höheren Konzentration des Antikoagulans als andere Teile des Bluts ausgesetzt wird.

2. Verfahren nach Anspruch 1, wobei die Tiefe jedes Durchflußkanals geringer als die Dicke der Tablette ist.

3. Verfahren nach Anspruch 1 oder 2, wobei die Dicke der Tablette (18) in der Weise vorgegeben ist, daß die Tablette bis zu dem Zeitpunkt nicht vollständig wegerodiert ist, in dem das letzte Blut, das die Durchflußkanäle entlanggeleitet wird, über die Tablette geströmt ist.

4. Verfahren nach Anspruch 1, 2 oder 3, wobei die Kammerwand Vorsprünge (20) definiert, die in Richtung der Tablette (18) vorspringen.

5. Verfahren nach einem der vorhergehenden Ansprüche, wobei das Blut in einem Behälter (12) gesammelt wird, der bis auf das Antikoagulans, das mit dem Blut in den Behälter strömt, im wesentlichen frei von dem Antikoagulans ist.

6. Verfahren nach einem der vorhergehenden Ansprüche, wobei das Antikoagulans ein geeignetes Blutkonservierungspräparat ist.

7. Verfahren nach Anspruch 6, wobei die Tablette (18) eine feste Dextrosemasse ist, die eine wirksame gerinnungshemmende Menge von löslichem, nichttoxischem Citrat enthält.

8. Verfahren nach Anspruch 6 oder 7, wobei die Tablette (18) eine Menge von löslichem, nichttoxischem Phosphat aufweist, die wirksam ist, um die Wirkung des Blutkonservierungs-Antikoagulans zu fördern.

9. Verfahren nach einem der vorhergehenden Ansprüche, wobei die Tablette (18) in Form von haftenden Teilchen vorliegt und Mannitol in ausreichender Menge enthält, um das Haften der Teilchen aneinander zu fördern.

10. Verfahren nach einem der vorhergehenden Ansprüche, wobei das Blut Vollblut ist.

11. Set zur Verwendung bei der Durchführung des Verfahrens nach Anspruch 1, das eine Leitung mit einer Kammer (16) aufweist, die ein Blut-Antikoagulans (18) enthält, dadurch gekennzeichnet, daß das Antikoagulans in Tablettenform (18) vorgesehen ist, wobei die Tablette gegenüberliegende ebene Hauptflächen mit größeren Dimensionen als die Dicke der Tablette hat, und daß die Tablette in der Kammer (16) angeordnet ist, um Blutdurchflußkanäle zwischen den Hauptflächen und der Kammerwand zu bilden.

12. Set nach Anspruch 11, wobei die Tiefe jedes Durchflußkanals geringer als die Dicke der Tablette ist.

13. Set nach Anspruch 11 oder 12, wobei die Kammerwand Vorsprünge (20) bildet, die in Richtung der Tablette (18) vorspringen.

14. Set nach Anspruch 11, 12 oder 13, wobei die feste Masse Dextrose aufweist, die eine wirksame gerinnungshemmende Menge von Antikoagulans enthält.

15. Set nach einem der Ansprüche 11 bis 14, wobei die feste Masse in Form von haftenden Teilchen vorliegt und Mannitol in ausreichender Menge enthält, um das Haften der Teilchen aneinander zu fördern.

16. Set nach einem der Ansprüche 11 bis 15, das eine Einrichtung (14) zur Verbindung mit dem Venensystem eines Blutspenders an einem Leitungsende hat.

17. Set nach einem der Ansprüche 11 bis 16, das an einen Blutkonservierungsbeutel (12) am anderen Leitungsende angeschlossen ist.

18. Set nach einem der Ansprüche 11 bis 17, wobei das Antikoagulans ein lösliches, nichttoxisches Citrat enthält.

19. Set nach einem der Ansprüche 11 bis 17, wobei das Antikoagulans Heparin enthält.

20. Set nach Anspruch 18, wobei die Tablette (18) eine Menge von löslichem, nichttoxischem Phosphat aufweist, die wirksam ist, um die Wirkung des Blutkonservierungs-Antikoagulans zu fördern.

21. Set nach einem der Ansprüche 11 bis 20, das ein Leitungsende hat, das an einen Blutkonservierungsbeutel (12) angeschlossen ist, wobei der Beutel im wesentlichen frei von einem gerinnungshemmenden Präparat ist.

**Revendications**

1. Procédé d'addition d'un agent anticoagulant dans du sang pendant une collecte sur un donneur, comprenant le passage du sang dans une chambre (16) d'un conduit à travers une réserve d'agent anticoagulant (18) soluble dans le sang dans ladite chambre, caractérisé en ce que ledit agent anticoagulant est fourni sous la forme d'un comprimé (18), le comprimé ayant des surfaces principales opposées planes qui ont une dimension plus grande que l'épaisseur du comprimé, en ce que le comprimé est disposé dans ladite chambre (16) de manière à former des passages d'écoulement entre lesdites surfaces principales et la paroi de chambre, et en ce que le sang passe le long desdits passages d'écoulement à travers lesdites surfaces principales, grâce à quoi une quantité contrôlée d'agent est forcée d'entrer dans ledit sang passant, grâce à quoi pratiquement aucune portion dudit sang n'est exposée à une concentration dudit agent sensiblement plus élevée que dans les autres portions dudit sang.

2. Procédé selon la revendication 1, dans lequel la profondeur de chaque passage d'écoulement est plus petite que l'épaisseur du comprimé.

3. Procédé selon la revendication 1 ou 2, dans lequel l'épaisseur du comprimé (18) est telle qu'il n'est pas complètement érodé avant que la partie restante du sang passant le long desdits passages d'écoulement soit passée sur le comprimé.

4. Procédé selon la revendication 1, 2 ou 3, dans lequel ladite paroi de chambre comporte des saillies (20) dirigées vers le comprimé (18).

5. Procédé selon une quelconque des revendications précédentes, dans lequel ledit sang est recueilli dans un conteneur (12) qui est essentiellement exempt dudit agent, sauf l'agent passant dans ledit conteneur avec ledit sang.

6. Procédé selon une quelconque des revendications précédentes, dans lequel ledit agent anticoagulant est une préparation convenant pour le stockage du sang.

7. Procédé selon la revendication 6, dans lequel ledit comprimé (18) est une masse solide de dextrose qui contient une quantité anticoagulante efficace de citrate non-toxique soluble.

8. Procédé selon la revendication 6 ou 7, dans lequel ledit comprimé (18) contient une quantité de phosphate non-toxique soluble efficace pour stimuler l'action dudit agent anticoagulant pour le stockage du sang.

9. Procédé selon une quelconque des revendications précédentes, dans lequel ledit comprimé (18) est sous la forme de particules adhérentes et contient du mannitol en une quantité suffisante pour stimuler l'adhésion desdites particules entre elles.

10. Procédé selon une quelconque des revendications précédentes, dans lequel ledit sang est du sang complet.

11. Appareil pour la mise en oeuvre du procédé de la revendication 1, comprenant un conduit ayant une chambre (16) qui contient un agent anticoagulant (18) pour le sang, caractérisé en ce que l'agent anticoagulant est fourni sous la forme d'un comprimé (18), ledit comprimé ayant des surfaces principales opposées planes d'une dimension plus grande que l'épaisseur du comprimé et en ce que le comprimé est disposé dans ladite chambre (16) de manière à former des passages d'écoulement pour le sang entre lesdites surfaces principales et la paroi de chambre.

12. Appareil selon la revendication 11, dans lequel la profondeur de chaque passage d'écoulement est plus petite que l'épaisseur du comprimé.

13. Appareil selon la revendication 11 ou 12, dans lequel ladite paroi de chambre comporte des saillies (20) dirigées vers le comprimé (18).

14. Appareil selon la revendication 11, 12 ou 13, dans lequel ladite masse solide comprend du dextrose qui contient une quantité d'anticoagulant efficace.

15. Appareil selon une quelconque des revendications 11 à 14, dans lequel ladite masse solide est sous la forme de particules adhérentes et contient du mannitol en une quantité suffisante pour stimuler l'adhésion desdites particules entre elles.

16. Appareil selon une quelconque des revendications 11 à 15, comportant un moyen (14) à une extrémité du conduit pour communiquer avec le système veineux d'un donneur de sang.

17. Appareil selon une quelconque des revendications 11 à 16, relié à une poche (12) de stockage de sang par l'autre extrémité du conduit.

18. Appareil selon une quelconque des revendications 11 à 17, dans lequel ledit agent anticoagulant contient un citrate non-toxique soluble.

19. Appareil selon une quelconque des revendications 11 à 17, dans lequel ledit agent anticoagulant contient de l'héparine.

20. Appareil selon la revendication 18, dans lequel ledit comprimé (18) contient une quantité de phosphate non-toxique soluble qui est efficace pour stimuler l'action dudit agent anticoagulant pour le stockage du sang.

21. Appareil selon une quelconque des revendications 11 à 20, comportant une extrémité de conduit reliée à une poche (12) de stockage de sang, dans lequel la poche est essentiellement exempte de préparation anticoagulante.

FIG. 1

FIG. 2